# EUROPEAN PATENT SPECIFICATION

(11) **EP 0 384 380 B1**
(45) Date of publication and mention of the grant of the patent: **19.06.1996**
(21) Application number: 90103210.2
(22) Date of filing: 20.02.1990
(51) Int. Cl.: C07K 5/06, C07K 5/08, A61K 38/08

(54) **Novel peptide derivatives and their pharmaceutical use**
Peptidderivate und deren pharmazeutische Verwendung
Dérivés peptidiques et leur utilisation pharmaceutique

(30) Priority: 21.02.1989 JP 39457/89; 02.05.1989 JP 112244/89; 02.11.1989 JP 284803/89; 15.11.1989 JP 294836/89; 15.12.1989 JP 323782/89
(43) Date of publication of application: 29.08.1990
(73) Proprietor: JAPAN TOBACCO INC., Tokyo 105 (JP)
(72) Inventor: Uchida, Itsuo, c/o Pharmaceutical Research Lab., Yokohama-shi, Kanagawa 227 (JP); Akira, Saito, c/o Pharmaceutical Research Lab., Yokohama-shi, Kanagawa 227 (JP); Yasuda, Akihiro, c/o Pharmaceutical Research Lab., Yokohama-shi, Kanagawa 227 (JP); Hara, Katsuyoshi, c/o Pharmaceutical Research Lab, Yokohama-shi, Kanagawa 227 (JP); Haruta, Junchi, c/o Pharmaceutical Research Lab, Yokohama-shi, Kanagawa 227 (JP); Anami, Koretake,, Nakatsu-shi, Oita 871 (JP); Iwata, Kunio, c/o Toxicology Res. Lab., Kanagawa 257 (JP); Matsushita, Mutsuyoshi, c/o Toxicol. Res. Lab. of, Kanagawa 257 (JP); Hari, Hiroaki, c/o Toxicology Res. Lab. of, Kanagawa 257 (JP); Furukawa, Noburu, c/o Toxicology Res. Lab. of, Kanagawa 257 (JP)
(74) Representative: von Kreisler, Alek, Dipl.-Chem.

(56) References cited:
- EP-A- 0 171 159
- CHEMICAL ABSTRACTS, vol. 92, no. 5, 4th February 1980, pages 825-826, abstract no. 42368f, Columbus, Ohio, US; L.G. BAUCE et al.: "Synthesis of a series of residue 1 (pyroglutamic acid) analogs of thyrotropin releasing hormone", & INT. J. PEPT. PROTEIN RES. 1979, 14(3), 216-26

## Description

### BACKGROUND OF THE INVENTION

This invention relates to peptide derivatives of the following general formula [I] and acid addition salts which exhibit more potent stimulant activities in central nerve system such as an antagonistic action against hypothermia, a locomoter stimulant action, a spinal reflex stimulant action, etc., as compared with other pharmaceuticals, particularly TRH (thyrotropin releasing hormone) and its derivatives, and thus are useful as a therapeutic medicament for central nervous disorders: 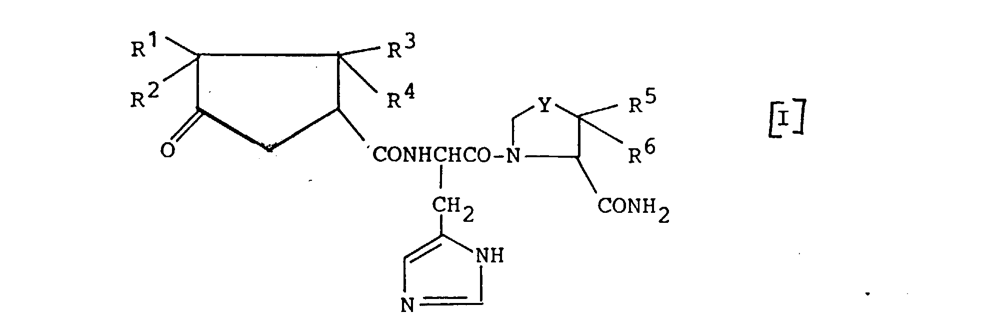 wherein R¹ and R² are the same or different and respectively mean a hydrogen atom or a C_{1 -5} alkyl group, R³ and R⁴ are the same or different and respectively mean a hydrogen atom or a C_{1 -5} alkyl or phenyl, or R³ and R⁴ combinedly mean a C₂₋₇ alkylene group, R⁵ and R⁶ are the same or different and respectively mean a hydrogen atom or a C_{1 -5} alkyl group, and Y means -CH₂- or -S-, or an acid addition salt thereof.

As the compounds relevant to the objective compounds [I] of the present invention, known is L-pyroglutamyl-L-histidyl-L-prolinamide (pGlu-His-Pro-NH₂) referred to as TRH (thyrotropin releasing hormone). TRH is known to have TSH (thyrotropin: thyroid stimulating hormone)-releasing activity and is also known to be useful as a therapeutic medicine for disturbance of consciousness caused by cerebral function disturbances. However, TRH has defects such that it is poorly stable in vivo, easily decomposed by pyroglutamylpeptidase, TRH amidase and other enzymes [Progress of Medicines (Igaku no Ayumi), vol. 134, No. 4, p. 252] and deactivated in a short period, necessitating frequent administration when used, which results in excessive secretion of TSH. For the improvement of the above-mentioned defects of TRH, studies from various aspects have been conducted for TRH derivatives which on the one hand reduce TSH secretion-inducing action as the side effect and on the other hand possess more potent central nerve stimulant actions such as an antihypnotic action, an anti-reserpine action (antagonistic action against hypothermia), a locomoter stimulant action, a spinal reflex stimulant action, a dopamine potentiating action and an anesthesia antagonism, and have more excellent durability in action than TRH. For example, the following reports have been presented:
US 4100152, Japanese Patent Unexamined Publication No. 33197/1986, No. 22797/1987 and No. 172996/1985, EP-.A-0 001 845, EP-A-0 171 159, GB 1564078, US 4788179, US 4636567 and US 4610821.

Further, Int. J. Pept. Protein Res. 14(3), 216-226 mentions among other compounds a TRH analogue of the following formula: 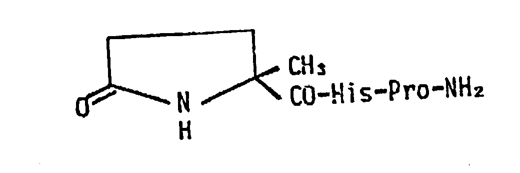 and the following compound:

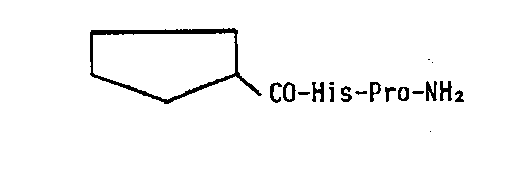

### SUMMARY OF THE INVENTION

This invention has been made to improve the above-mentioned defects of TRH, and the object of the present invention is to provide novel peptide derivatives and acid addition salts thereof which are useful as a therapeutic medicine for central nervous disturbances, and which possess more potent stimulant actions on central nervous system and long durability than TRH and other known TRH derivatives and pharmaceutical use thereof.

According to the present invention, the novel peptide derivatives and acid addition salts thereof are represented by the following general formula [I] 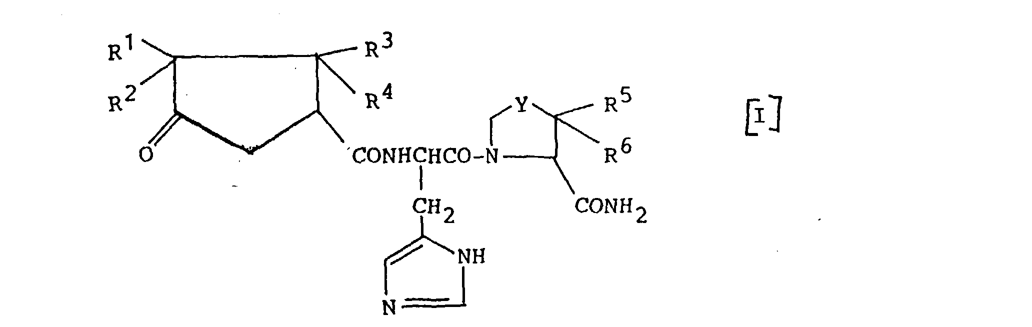 wherein R¹ and R² are the same or different and respectively mean a hydrogen atom or a C₁₋₅ alkyl group, R³ and R⁴ are the same or different and respectively mean a hydrogen atom or a C₁₋₅ alkyl or phenyl, or R³ and R⁴ combinedly mean a C₂₋₇ alkylene group, R⁵ and R⁶ are the same or different and respectively mean a hydroqen atom or a C₁₋₅ alkyl group,
and Y means -CH₂- or -S-, or an acid addition salt thereof.

Preferred are, among others, the derivatives of the general formula [I ], wherein R¹ and R² are a hydrogen atom.

### DETAILED DESCRIPTION OF THE INVENTION

In the present specification, the C₁₋₅ alkyl group means a straight or branched carbon-chain having, preferably, one through four carbon atoms, which is exemplified by methyl group, ethyl group, propyl group, butyl group, isopropyl group and sec-butyl group. The C₂₋₇alkylene group means a bivalent straight aliphatic saturated hydrocarbon having two through seven carbon atoms, preferably two through five carbon atoms, which includes, for example, ethylene group, trimethylene group, tetramethylene group, pentamethylene group, hexamethylene group and heptamethylene group. The alkylene group taken together with the adjacent carbon atom forms a spirocycloalkane.

With regard to TRH derivatives, various studies have been conducted as mentioned above, and for example, there have been known TRH derivatives in which the pGlu-moiety of TRH is converted into a heterocyclic group such as

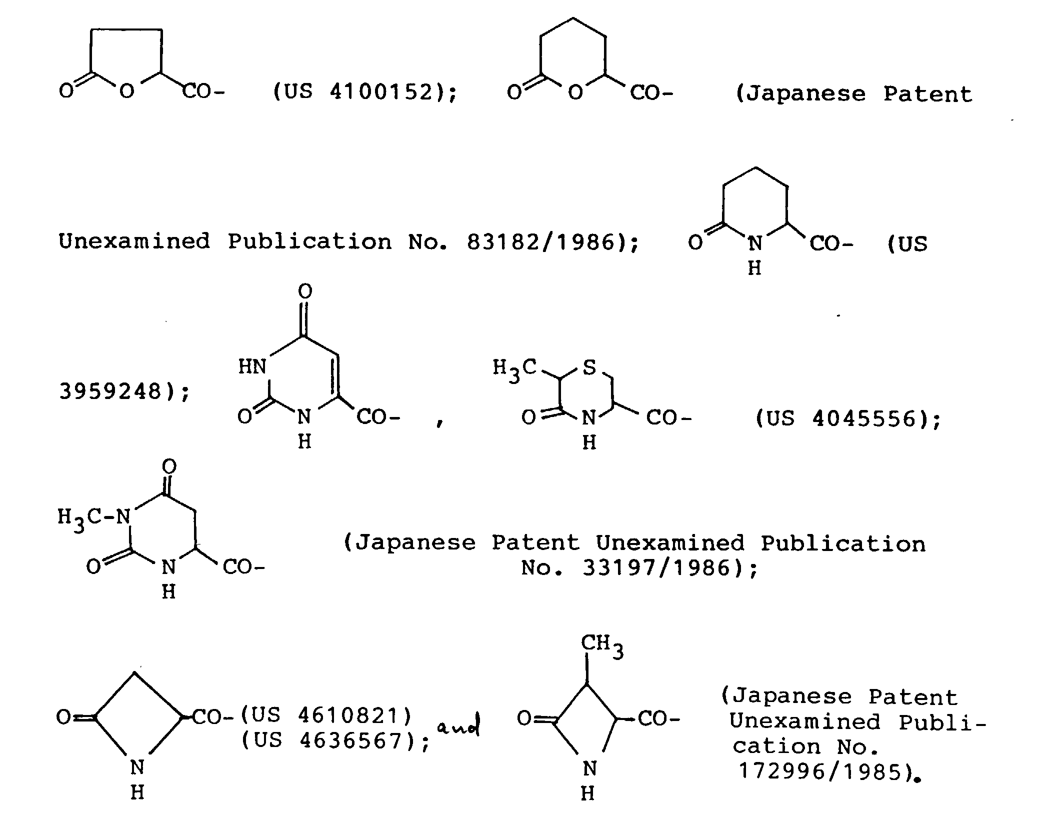

Heretofore, however, there have not been made any attempt to convert the pGlu-moiety of TRH into a structure of the general formula [II] 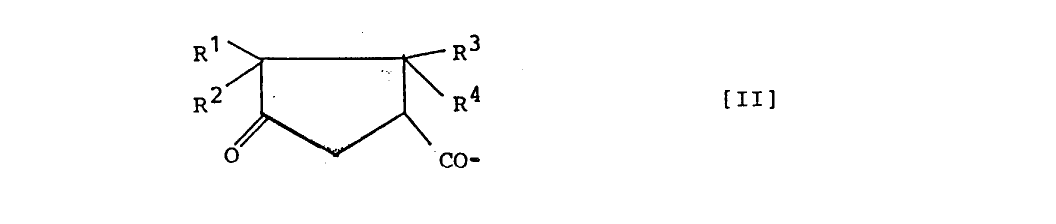 wherein R¹, R², R³ and R⁴ are of the same meaning as defined above.

This invention is characterized by the above-mentioned chemical structure, and the conversion into this characteristic structure resulted in enabling of provision of the novel TRH derivatives possessing more potent central nerve stimulant actions and more lasting pharmacological effects than TRH or other known derivatives thereof.

The objective compounds [I] of this invention are capable of forming salts thereof with an acid. Examples of the acid include inorganic salts such as hydrochloric acid and sulfuric acid or organic salts such as citric acid, acetic acid and tartaric acid. These salts are also encompassed in the present invention.

The objective compounds [I] of the present invention have at least two asymmetric carbons, and there exist stereoisomers based on such asymmetric carbons. The objective compounds of the present invention include respective isolated stereoisomers and the mixtures of the stereoisomers.

According to the present invention, the objective compounds [I] or their acid addition salts can be produced by the following method:
(A) Condensing a compound of the general formula [III] 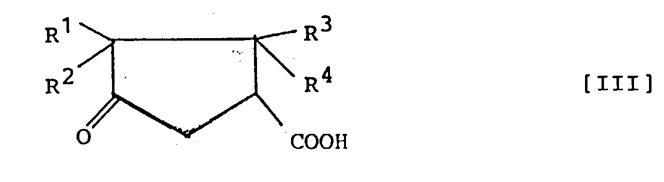 wherein R¹ - R⁴ are of the same meaning as defined above, or a reactive derivative thereof with a compound of the general formula [IV] 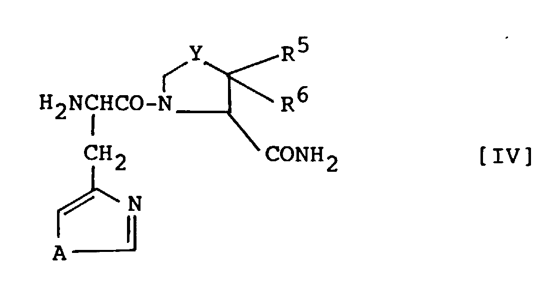 wherein A means imino group or a protected imino group, R⁵, R⁶ and Y are of the same meaning as defined above or a salt thereof;
(B) Condensing a compound of the general formula [V] 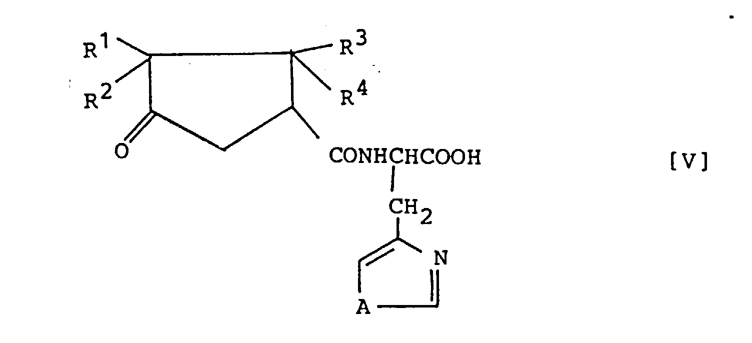 wherein R¹ - R⁴ and A are of the same meaning as defined above, or a salt thereof, or a reactive derivative thereof with an amino acid amide of the general formula [VI] 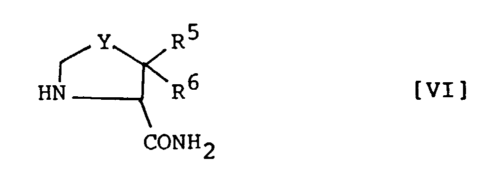 wherein Y, R⁵ and R⁶ are of the same meaning as defined above, or a salt thereof; or
(C) subjecting a compound of the general formula [VII] 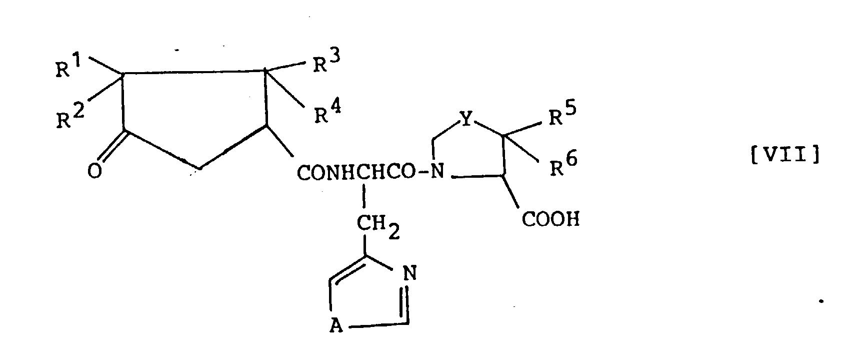 wherein R¹ - R⁶, A and Y are of the same meaning as defined above, or a salt thereof, or a reactive derivative thereof to amidation, and (D) thereafter, when A in the product thus-obtained by the above step (A), (B) or (C) is a protected imino group, removing the protective group; and
(E) whereafter if desired, converting the thus-obtained product into an acid addition salt thereof.

The starting compounds [IV] - [VII] of the present invention can be subjected to the respective reaction either in the form of free base or in the form of salt. The salt can be preferably used in the form of acid addition salt, and as such acid addition salt, use can be made of, for example, an inorganic acid addition salt such as hydrochloride or hydrobromide, or an organic acid addition salt such as trifluoroacetate or p-toluenesulfonic acid.

As the reactive derivative of the compounds [III], [V] and [VII], use can be made of an active ester (e.g. N-hydroxysuccinimido ester, pentachlorophenyl ester, N-hydroxybenzotriazole ester), an acid halide (e.g. acid chloride), an acid azido, an acid anhydride and imidazolamide, corresponding to the respective compounds. The active ester may be subjected to peptide-synthesizing reaction after isolated or without being isolated. As the reactive derivative of the compound [VII], use can be preferably made of, for example, an ester (an alkyl ester such as ethyl ester or methyl ester; and an aralkyl ester such as benzyl ester.

When the group A in the above-mentioned starting compounds [IV], [V] and [VII] is a protected imino group, use can be made of any protective group conventionally used in peptide synthesis as the protective group of the imino group. Examples of the suitable protective group include benzyloxycarbonyl group, benzyl group, tosyl group, tert-butoxycarbonyl group and 2,4-dinitrophenyl group.

### [Reaction Steps (A) and (B)]

Both of the condensation reaction of a compound [III] or a reactive derivative thereof with a compound [IV] or a salt thereof and the condensation reaction of a compound [V] or a salt thereof or a reactive derivative thereof with a compound [VI] or a salt thereof, which are peptide synthetic reactions, can be carried out by a conventional means. For example, the condensation reaction of a compound [III] with a compound [IV] or a salt thereof and that of a compound [V] or a salt thereof with a compound [VI] or a salt thereof can be carried out in a suitable solvent in the presence of a condensing agent. Examples of the suitable condensing agent include dicyclohexylcarbodiimide, 1-ethyl-3-(3-dimethylaminopropyl)carbodiimide, phosphorus oxychloride, phosphorus trichloride, thionyl chloride, oxalyl chloride, triphenylphosphine and Vilsmeier Reagent. The condensation reaction is carried out preferably at -50 - 50°C. When a salt of a compound [IV] is used, the reaction is conducted in the presence of a deacidifying agent.

On the other hand, the condensation reaction of a reactive derivative of a compound [III] with a compound [IV] or a salt thereof and that of a reactive derivative of a compound [V] with a compound [VI] or a salt thereof can be carried out in a suitable solvent in the presence or absence of a deacidifying agent. As the deacidifying agent, mention can be made of, for example, trialkylamines (triethylamine and trimethylamine ), N,N-dialkylanilines (N,N-dimethylaniline and N,N-diethylaniline), pyridine, N-alkylmorpholines (N-methylmorpholine, etc.), alkali metal hydroxides (potassium hydroxide and sodium hydroxide), alkali metal carbonates (potassium carbonate), and alkali metal hydrogencarbonates (sodium hydrogencarbonate). This condensation reaction can be suitably carried out at -50-50°C, particularly -10 - 10°C.

As the solvent for the condensation reaction usable in the reaction steps (A) and (B), use can be made of, for example, N,N-dimethylformamide, dichloromethane, dioxane, tetrahydrofuran, acetonitrile, ethyl acetate, pyridine, acetone and water.

### [Reaction Step (C)]

The amidation reaction of a compound [VII], a salt thereof or a reactive derivative thereof can be carriea out by reacting the compound with ammonia or an ammonia-donating compound. For example, the amidation reaction of a compound [VII] or a salt thereof with ammonia or an ammonia-donating compound can be preferably carried out in the presence of a dehydrating agent in a suitable solvent. As the dehydrating agent, use can be made of, for example, any compound mentioned above as the suitable condensing agent. As the ammonia-donating compound, use can be made of any compound capable of releasing ammonia in the reaction mixture, which is exemplified by ammonium chloride and ammonium carbonate. The amidation reaction is preferably carried out at -20°C - 20°C. As the suitable solvent, mention can be made of, for example N,N-dimethylformamide, dimethylsulfoxide and tetrahydrofuran.

Furthermore, the amidation reaction of a reactive derivative of a compound [VII] or a salt thereof with ammonia or an ammonia-donating compound can be carried out in the presence or absence of a deacidifying agent in a suitable solvent. As the deacidifying agent, use can be made of any of the deacidifying agents mentioned in the description of the above reaction steps (A) and (B). The amidation reaction is preferably carried out at -20°C - 20°C. As the suitable solvent, mention can be made of methanol, ethanol, dimethylformamide and dimethylsulfoxide.

### [Reaction Step D]

When A in the compound produced in the above-mentioned reaction step (A), (B) or (C) are (is) a protected imino, the deprotection of the protective group can be carried out easily, depending upon the species of the protective group, in accordance with a conventional method such as catalytic reduction, electrolytic reduction, acid treatment, base treatment or oxidation reaction.

### [Reaction Step (E)]

The thus-obtained objective compound can be, if necessary, converted into an acid addition salt easily by treating with a stoichiometric amount of an acid in accordance with a conventional method.

In the above-mentioned reactions, the staring compounds [III] - [VII], the intermediate products and the objective compounds contain 1-6 asymmetric carbon atom(s), and the above methods of the present invention can be carried out using the respective optically active isomers of the starting compounds [III] - [VII] or their respective mixture. Since the above-mentioned reactions of the present invention can be carried out without involving racemization, the intermediate products and the objective compounds can be obtained in the form of the corresponding optically active isomer when the respective optically active isomers of the starting compounds [III] - [VII] are used.

The objective compounds [I] and acid addition salts thereof can be administered orally or nonorally as they are or in the form of, for example, powders, granules, tablets, capsules, injections (intravenous, subcutaneous, intramuscular) or suppositories suitably in admixture with pharmacologically acceptable carriers, excipients and diluents.

While the dosage of the objective compounds [I] or their acid addition salts of the present invention varies depending on the administration route, the age, body weight or symptom of the patient, generally it is preferably 0.5 µg - 5 mg/kg/day. Particularly, in the case of oral administration, the dosage is preferably 10 µg - 5 mg/kg/day and in the case of parenteral administration (e.g. intravenous administration, intramuscular administration, subcutaneous administration), the dosage is preferably 1 - 1000 µg/kg/day.

Since the compounds [I] or their acid addition salts of the present invention possess more potent actions on central nervous system (e.g. antagonistic action against hypothermia, locomotor stimulant action, spinal reflex stimulant action), they are of use as a therapeutic medicament for central nervous disorders such as impaired consciousness, depression and hypomnesia, in association with schizophrenia, melancholia, senile dementia, sequelae of cerebrovascular disorders, head trauma, epilepsy, and spinocerebellar degeneracy.

Below, the present invention is in further detail described by illustrating reference examples and working examples. The abbreviations used in the examples stand for the following meanings respectively.
- NMR: nuclear magnetic resonance spectra (¹H-NMR)
- SIMS: secondary ion mass spectra
- EIMS: electronimpact mass spectra
- CIMS: chemical ionization mass spectra
- DMF: N,N-dimethylformamide
- HOBT: 1-hydroxy-1,2,3-benzotriazole
- DCC: dicyclohexylcarbodiimide
- THF: tetrahydrofuran
- WSC: 1-ethyl-3-(3-dimethylaminopropyl)carbodiimide

The compound which contains the symbol N^{im} in the compound name is the mixture of the compound substituted at the π nitrogen atom 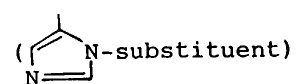 and the compound substituted at the τ nitrogen atom

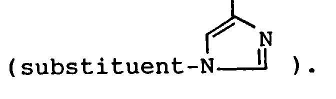

### Reference Example 1

### cis-2-Methyl-4-oxocyclopentanecarboxylic acid

In acetonitrile (5 ml) were dissolved methyl cis-2-methyl-4-oxocyclopentanecarboxylate [K. Kojima et al., Chem. Pharm. Bull., 33, 2750 (1985)] (827 mg) and sodium iodide (3.175 g), and trimethylchlorosilane (2.69 ml) was added to the solution at room temperature. The mixture was refluxed under nitrogen atmosphere for one day. After the temperature was cooled to room temperature, water was added and the mixture was extracted with ether, followed by washing with an aqueous solution of sodium thiosulfate. The water layer was further extracted with ether. The organic layers were combined, dried over magnesium sulfate and concentrated tb dryness. The residue was subjected to silica gel column chromatography with ethyl acetate : chloroform (1:1), and the eluted fractions were combined and concentrated to dryness to yield the title compound (243 mg).
NMR (CDCl₃)
δppm: 1.14 (3H, d, J=7.0Hz), 2.18 (1H, ddd, J=18.3Hz, 7.8Hz, 1.6Hz), 2.34 - 2.47 (2H, m), 2.63 (1H, ddd, J=18.3Hz, 6.0Hz, 1.6Hz), 2.74 (1H, m), 3.23 (1H, m)

### Reference Example 2

### trans-2-Methyl-4-oxocyclopentanecarboxylic acid

Methyl trans-2-methyl-4-oxocyclopentanecarboxylate [K. Koj ima et at., Chem. Pharm. Bull., 33, 2750 (1985)] (495 mg) was dissolved in methanol (3 ml), and a 1N aqueous solution of sodium hydroxide (3.5 ml) was added to the solution at 0°C. After the mixture was stirred at room teperature for 1 hour, it was acidified with dilute hydrochloric acid, followed by extraction with ether. The extract was concentrated to dryness and the residue was subjected to silica gel column chromatography with ethyl acetate-chloroform (1:1) as the eluent, and the eluted fractions were combined and concentrated to dryness to afford the title compound (219 mg) as crystals.
NMR (CDCl₃)
δppm: 1.26 (3H, d, J=6.4Hz), 1.94 (1H, m), 2.45 - 2.65 (4H, m), 2.74 (1H, m)

### Reference Example 3

### 1,4,4-Tricarboethoxy-3-oxospiro[4.5]decane

Diethyl cyclohexylidenemalonate [W. Lehnert, Tetrahedron, 29, 635 (1973)] (3.1 g) and 1,4-bis(trimethylsiloxy)-1,4-diethoxy-1,3-butadiene [N.R. Long et al, Synthetic Commun., 11, 687 (1981)] (5 g) were dissolved in dry dichloromethane (40 ml), and 15.7 ml of a 1.0 M solution of titanium (IV) chloride in dichloromethane was added to the solution at room temperature under nitrogen atmosphere. After the mixture was stirred at room temperature for 8 hours, 25 ml of a 10% aqueous solution of sodium hydrogen carbonate was added and the organic layer was separated, dried over magnesium sulfate and concentrated to dryness. The residue was subjected to silica gel column chromatography with hexane-ethyl acetate (5:1) as the eluent, and the eluted fractions were combined and concentrated to dryness to afford the title compound (990 mg).
NMR (CDCl₃)
δppm : 1.18 - 1.37 (9H, m), 1.42 - 1.82 (10H, m), 2.62 (1H, dd, J=8.7Hz and 19.1Hz), 3.01 (1H, dd, J=4.9Hz and 19.1Hz), 3.36 (1H, dd, J=4.9Hz and 8.7Hz), 4.07 - 4.32 (6H, m)
IRν (CHCl₃) cm⁻¹ : 1771 and 1726

### Reference Example 4

### 1-Carboxy-3-oxospiro[4.5]decane

1,4,4-Tricarboethoxy-3-oxospiro[4.5]decane (990 mg), water (196 µl) and lithium chloride (350 mg) were dissolved in dimethylsulfoxide (25 ml), and the mixture was stirred at 170°C for 4 hours. After cooling to room temperature, saturated brine (40 ml) was added and the mixture was extracted with ethyl acetate. The organic layer was separated, dried over magnesium sulfate and concentrated to dryness. The residue was purified through silica gel column chromatography (hexane-ethyl acetate = 5:1) to give pure 1-carboethoxy-3-oxospiro[4.5]decane (450 mg), which was added to 5N hydrochloric acid (20 ml), and the mixture was refluxed for 3 hours. After cooling to room temperature, the mixture was concentrated and the residue was adjusted to pH 10 with aqueous solution of sodium hydroxide, followed by washing with ether. The aqueous layer was acidified with 5N hydrochloric acid to pH 2, followed by extraction with ether. The ethereal solution was dried, and concentrated to dryness to afford the title compound (310 mg) as colorless crystals.
m.p. 146 - 147°C
NMR (CDCl₃)
δppm: 1.20 - 1.80 (10H, m), 2.15 (1H, d, J=18.2Hz), 2.41 - 2.71 (3H, m), 2.93 (1H, dd, J=6.1Hz and 8.0Hz)

### Reference Example 5

### N^{α}-(cis-3-Methylpyroglutamyl)-L-histidyl-L-prolinamide

L-Histidyl-L-prolinamide dihydrobromide (4.33 g) was dissolved in DMF (15 ml), and triethylamine (2.92 ml) was added to the solution under cooling at -10°C. After the mixture was stirred under ice-cooling for 10 minutes, the resulting precipitation was filtered off to give a DMF solution of L-histidyl-L-prolinamide, which was immediately used in the following synthetic reaction.

cis-3-Methylpyroglutamic acid [A.B. Mauger, J. Org. Chem., 46, 1032 (1981)] (1.5 g) was dissolved in DMF (30 ml), and HOBT (1.6 g) was added to the solution, followed by addition of DCC (2.81 g) under cooling at 0°C. The mixture was stirred at the same temperature overnight. The above-mentioned DMF solution of L-histidyl-L-prolinamide was added to the mixture, and the mixture was stirred at 5°C overnight. After the resulting precipitation was filtered off, the filtrate was concentrated to dryness under reduced pressure and the residue was subjected to silica gel column chromatography with chloroform-methanol-ammonia water (40:10:1) as the eluent. The eluted fractions were combined and concentrated to dryness to give N^{α}-(cis-3-methylpyroglutamyl)-L-histidyl-L-prolinamide (2.2 g) as powders.
NMR (CD₃OD)
δppm: 0.88 (1.5H, d, J=7.0Hz), 0.96 (1.5H, d, J=7.0Hz), 1.80 - 2.16 (4H, m), 2.16 - 2.47 (2H, m), 2.78 (1H, m), 3.09 (1H, m), 3.18 (1H, m), 3.52 (1H, m), 3.88 (1H, m), 4.22 (1H, m), 4.44 (1H, m), 4.90 - 5.00 (1H), 7.18 (1H, s), 8.04 (0.5H, s), 8.09 (0.5H, s)
SIMS m/z: 377 (M+1)⁺

### Reference Example 6

### 3-[N^{α}-(cis-3-Methylpyroglutamyl)-L-histidyl]-L-thiazolidine-4-carboxamide

3-(N^{im}-Tosyl-L-histidyl)-L-thiazolidine-4-carboxamide hydrochloride [Itsuro Sobue, et al, Japanese Patent Unexamined Publication No. 190795/1985] (500 mg), cis-3-methylpyroglutamic acid (234 mg) and HOBT (249 mg) were dissolved in a mixture of DMF (5 ml) and THF (5 ml), and WSC. hydrochloride (230 mg) was added to the solution while stirring under cooling at -10°C. Thereto was added triethylamine (227 µl), and after the pH was adjusted to pH 5, the mixture was stirred at room temperature for 2 days. After the resultant red at room temperature for 2 days. After the resultant precipitation was filtered off, the filtrate was concentrated under reduced pressure to dryness. The residue was subjected to purification by HP-20 column (1.0 x 40 cm). Washing with water and eluting with a mixture of water-acetone (1:1) was conducted in order, and the eluted fractions were concentrated under reduced pressure to dryness, and the obtained residue was further purified by silica gel column chromatography with chloroform-methanol-ammonia water (40:10:1) as the eluent. From the fractions containing the objective compound, 3-[N^{α} -(cis-3-methylpyroglutamyl)-L-histidyl]-L-thiazolidine-4-carboxamide (120 mg) was obtained.
NMR (CD₃OD)
δppm: 0.83 - 1.10 (3H), 1.08 (1H, m), 2.35 (1H, m), 2.79 (1H, m), 3.01 (1H, m), 3.10 - 3.25 (2H, m), 3.32 (1H), 4.14 (1H, t, J=7.0Hz), 4.44 (1H, t, J=9.0Hz), 4.81 (1H, m), 4.94 (1H, m), 5.07 (1H, t, J=9.0Hz), 7.01 (1H, s), 7.73 (1H, s)
SIMS m/z: 395 (M+1)⁺

### Example 1

### N^{α}-(3-Oxocyclopentanecarbonyl)-L-histidyl-L-prolinamide (Compound 1)

By the same method as in Reference Example 5, the title compound (222 mg) was obtained as powders from 3-oxocyclopentanecarboxylic acid [K. Curry, M. J. Peet, D. S. K. Magunuson and H. McLennan, J. Med. Chem., 31, 864 (1988)] (158 mg) and L-histidyl-L-prolinamide dihydrobromide (510 mg). NMR (CD₃OD)
δppm: 1.73 - 2.11 (4H, m), 2.11 - 2.38 (6H, m), 2.97 (1H, dd, J=7Hz and 14Hz), 3.11 (2H, dd, J=7Hz and 14Hz), 3.30 - 3.41 (1H, m), 3.72 - 3.83 (1H, m), 4.43 (1H, dd, J=4Hz and 9Hz), 4.81 (1H, t, J=7Hz), 6.96 (1H, s), 7.62 (1H, s)
CIMS m/z: 362 (M+1)⁺

### Example 2

### N^{α} -[(1R)-3-Oxocyclopentanecarbonyl]-L-histidyl-L-prolinamide

### (Compound 2)

By the same method as in Reference Example 5, the title compound (222 mg) was obtained as powders from (1R)-3-oxocyclopentane-carboxylic acid [K. Toki et al, Bull. Chem. Soc. Jpn., 31, 333 (1958)] (158 mg) and L-histidyl-L-prolinamide dihydrobromide (510 mg).
NMR (CD₃OD)
δppm: 1.70 - 2.40 (10H, m), 2.96 (1H, dd, J=14.6Hz and 6.7Hz), 3.00 - 3.15 (2H, m), 3.33 (1H, m), 3.77 (1H, m), 4.43 (1H, dd, J=8.3Hz and 4.2Hz), 4.78 (1H, t, J=7.0Hz), 6.95 (1H, s), 7.62 (1H, s)

### Example 3

3-[N^{α}-(3-Oxocyclopentanecarbonyl)-L-histidyl]-L-thiazolidine-4-carboxamide (Compound 3) By the same method as Reference Example 6, the title compound (137 mg) was obtained as powders from 3-(N^{im}-tosyl-L-histidyl)-L-thiazolidine-4-carboxyamide hydrochloride (400 mg) and 3-oxocyclopentanecarboxylic acid (168 mg).
NMR (CD₃OD)
δppm. 1.83 - 2.42 (6H, m), 2.92 - 3.23 (5H, m), 4.37 (1H, t), 4.61 - 5.10 (3H, m), 6.98 (1H, s), 7.64 (1H, s) CIMS m/z: 380 (M+1)⁺

### Example 4

### N^{α}-[(1R)-3-Oxocyclopentanecarbonyl]-L-histidyl-3,3-dimethylprolinamide (Compound 4)

By the same method as in Reference Example 5, the title compound (214 mg) was obtained as powders from (1R)-3-oxocyclopentanecarboxylic acid [K. Toki et al, Bull. Chem. Soc. Jpn., 31, 333 (1958)] (120 mg) and L-histidyl-3,3-dimethylprolinamide 2 dihydrobromide [Ger. Offen. 2609154 (1976) and Japanese Patent Unexamined Publication No. 116465/1977 (1977)] (413 mg).
NMR (CD₃OD)
δppm : 0.88 (1.5H, s), 1.04 (1.5H, s), 1.10 (1.5H, s), 1.17 (1.5H, s), 1.58 (0.5H, m), 1.73 (0.5H, m), 1.80 - 2.10 (2H, m), 2.10 - 2.40 (5H, m), 2.85 - 3.15 (3H, m), 3.22 (0.5H, m), 3.48 (0.5H, m), 3.78 (0.5H, s), 3.84 (0.5H, m), 3.97 (0.5H, m), 4.02 (0.5H, s), 4.77 (1H, s), 6.88 (0.5H, s), 6.92 (0.5H, s), 7.61 (0.5H, s), 7.64 (0.5H, s)

### Example 5

### N^{α}-(cis-2-Methyl-4-oxocyclopentanecarbonyl)-L-histidyl-L-prolinamide (Compound 5)

By the same method as in Reference Example 5, the title compound (306 mg) was obtained from cis-2-methyl-4-oxocyclopentanecarboxylic acid (213 mg) and L-histidyl-L-prolinamide dihydrobromide acid (620 mg).
NMR (CD₃OD)
δppm: 0.86 (1.5H, d, J=7.0Hz).1.01 (1.5H, d, J=7.0Hz), 1.70 - 2.15 (5H, m), 2.15 - 2.50 (4H, m,), 2.60 (1H,m), 2.98 (1H, dd, J=14.0Hz and 6.9Hz), 3.08 (1H, m), 3.33-3.50 (1H, m), 3.83 (1H, m), 4.44 (1H, m), 4.83 (1H, m), 6.97 (0.5H, s), 7.00 (0.5H, s), 7.61 (1H, s)
CIMS m/z: 376 (M+1)⁺

### Example 6

### N^{α}-(trans-2-Methyl-4-oxocyclopentanecarbonyl)-L-histidyl-L-prolinamide (Compound 6)

By the same method as in Reference Example 5, the title compound (150 mg) was obtained from trans-2-methyl-4-oxocyclopentane-carboxylic acid (190 mg) and L-histidyl-L-prolinamide dihydrobromide (552 mg).
NMR (CD₃OD)
δppm: 1.02 (1.5H, d, J=7.0Hz), 1.13 (1.5H, d, J=7.0Hz), 1.70 - 2.10 (4H, m), 2.10 - 2.60 (5H, m), 2.67 (1H, m), 3.00 (1H, m), 3.15 (1H, m), 3.45 (1H, m), 3.89 (1H, m), 4.47 (1H, m), 4.85 (1H, m), 6.98 (0.5H, s), 7.02 (0.5H, s), 7.65 (1H, s)
CIMS m/z: 376 (M+1)⁺

### Example 7

### N^{α}-(2,2-Dimethyl-4-oxocyclopentanecarbonyl)-L-histidyl-L-prolinamide (Compound 7)

In DMF (2 ml) was dissolved 2,2-dimethyl-4-oxocyclopentanecarboxylic acid [W. H. Perkin, jun., J. F. Thorpe, J. Chem. Soc., 79, 729 (1901)] (100 mg), and HOBT (108 mg) was added to the solution, followed by addition of DCC (145 mg) under cooling at 0°C. The mixture was stirred at 5°C overnight. After addition of L-histidyl-L-prolinamide dihydrobromide (265 mg) followed by addition of triethylamine (0.134 ml), the mixture was stirred at 5°C for 4 days. After the resultant precipitation was filtered off, the filtrate was concentrated under reduced pressure to dryness and the residue was subjected to silica gel column chromatography. The fractions eluted by chloroform : methanol : ammonia water (90:10:2) were combined and concentrated to dryness to give the title compound (140 mg) was obtained as powders.
NMR (CD₃OD)
δppm : 0.88 (1.5H, s), 1.00 (1.5H, s), 1.10 (1.5H, s), 1.21 (1.5H, s), 1.65 - 2.30 (6H, m), 2.44 (2H, m), 2.82 (1H, m), 2.96 (1H, m), 3.10 (1H, dd, J=15.0Hz and 7.2Hz), 3.30 - 3.55 (1H, m), 3.82 (1H, m), 4.43 (1H, m), 4.83 (1H, m), 6.94 (0.5H, s), 6.98 (0.5H, s), 7.60 (0.5H, s), 7.61 (0.5H, s)

### Example 8

### N^{α}-(3-oxospiro[4.5]decane-1-carbonyl)-L-histidyl-L-prolinamide (Compound 8)

By the same method as in Reference Example 5, the title compound (220 mg) was obtained from 1-carboxy-3-oxospiro[4.5]decane (150 mg) and L-histidyl-L-prolinamide dihydrobromide (389 mg).
NMR (CD₃OD)
δppm : 1.09 - 1.71 (10H, m), 1.85 - 2.52 (8H, m), 2.72 - 3.01 (2H, m), 3.10 (1H, dd), 3.42 - 3.56 (1H, m), 3.74 - 3.92 (1H, m), 4.31 - 4.46 (1H, m), 6.93 (0.5H, s), 6.98 (0.5H, s), 7.60 (0.5H, s), 7.62 (0.5H, s)
SIMS m/z: 431 (M+2)⁺

Needless to say, the present invention is not limited to these examples, and, for example, the following compounds are also encompassed in the present invention.
9. 3-[N^{α}-(3-Oxocyclopentanecarbonyl)-L-histidyl]-L-5-methylthiazolidine-4-carboxamide
10. N^{α}-(4-Oxo-2-phenylcyclopentanecarbonyl)-L-histidyl-L-prolinamide
11. N^{α}(6-Oxospiro[2.4]heptane-4-carbonyl)-L-histidyl-L-prolinamide
12. 3-[N^{α}-(2-Methyl-4-oxocyclopentanecarbonyl)-L-histidyl]-L-thiazolidine-4-carboxamide

The evaluation tests for anti-reserpine action, anti-chlorpromazine action and spinal reflex stimulant action were conducted as regards the compounds of the present invention.

For comparison, the same tests were conducted also as regards TRH and the hitherto-known DN1417 of the following formula:

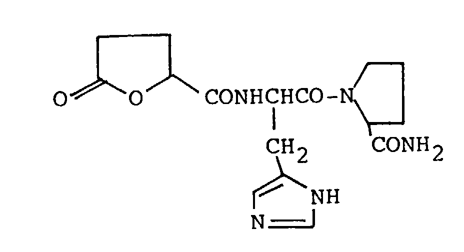

### Test Example 1

### Anti-reserpine action (Antagonistic action against hypothermia)

ICR male mice weighing 35 - 40 g were subcutaneously administered with reserpine at the dose of 5 mg/kg. About 2 hours later, each of the mice was held by a mouse-holder, and a body temperature sensor was intrarectally inserted. Thereafter, the body temperature was automatically measured every 10 minutes. The test compound dissolved in saline was subcutaneously administered 3 or 3.5 hours after administration of reserpine. After the administration of the test compound, the body temperature was measured every 10 minutes over the period of 120 minutes. The area below the body temperature line during the period was calculated, from which the regression line was estimated based upon least square method, and estimated was the amount of the test compound necessary for raising the body temperature by 1°C on the average for 120 minutes from that just before the administration of the test compound (Table 1).

**Table 1**

| | Concentration of test compound necessary for raising body temperature by 1°C on the average for 120 minutes (mg/kg, SC) |
|---|---|
| TRH | 2.52 |
| DN1417 | 2.06 |
| Compound 1 | 0.71 |
| Compound 2 | 0.12 |
| Compound 5 | 0.14 |
| Compound 6 | 0.27 |
| Compound 7 | 0.18 |

### Test Example 2

### Anti-chlorpromazine action (Locomotor stimulant action)

ICR male mice weighing 35 - 40 g (four animals per group) were subcutaneously administered with chlorpromazine HCl at the dose of 5 mg/kg and immediately set in the automatic locomotor-measuring apparatus (AUTOMEX). One hour later, the test compound dissolved in saline was subcutaneously administered, and the locomotor activity was measured every 15 minutes for 120 minutes. The total locomotor activity during the period of 120 minutes was calculated and the regression line was estimated based on the method of least squares, from which the amount of the test compound necessary for inducing 3000 locomotor-count was estimated (Table 2).

**Table 2**

| | Concentration of test compound necessary for inducing 3000 count of automatism quantity in 2 hours (mg/kg, SC) |
|---|---|
| TRH | 6.82 |
| DN1417 | 4.34 |
| Compound 1 | 1.03 |
| Compound 2 | 0.88 |
| Compound 5 | 0.54 |
| Compound 6 | 1.30 |
| Compound 7 | 0.83 |

### Test Example 3

### Flexor reflex in spinalized rats

Wistar male rats (weighing 450 - 520 g) were anesthetized with ether, and the cervical part of spinal cord was exposed, into which a tracheal cannula was inserted. The vagus nerves were severed bilaterally at the cervical region. Thereafter, the cervical vertebra C1 segment was transected, and rapidly, artificial ventilation was given with the use of an artificial respirator (Shinano Factory 60 rpm. 4 ml/stroke). The rat was placed on a thermostated apparatus in which water thermostated at 37°C was circulated and fixed thereto at forelimbs, right hindlimb, tooth and tail. A venous cannula was inserted into the femoral vein in the right hindlimb and two needle electrodes were subcutaneously inserted into the left skin of the toe, and a piece of thread was attached to the limb with the edge of the thread bonded to a force-displacement transducer. Tension of about 5 g was applied to the thread. Through the elctrodes, electric stimuli of 50 - 100 V were applied once per 30 seconds. The flexor reflex was recorded on the polygraph via a force-displacement transducer. After the reaction became constant in about one-hours' lapse for stabilization, the test compound dissolved in saline was intravenously administered. Thereafter, the reaction was recorded for 1 hour. In the data, taking the mean of the three reactions just before administration of the test compound for 100%, the reflex amplitude at 1-, 3-, 5-, 8-, 10-, 15-, 20-, 30-, 40-, 50- and 60- minutes' lapse after administration of the test compound were calculated. In Table 3, there are shown calculated area below the straight line linking the obtained reflex amplitude at the respective above-mentioned time covering 60 minutes in the case of administration of each test compounds at the dose of 0.1 mg/kg, and the reflex amplitude at 10- and 60-minutes' lapse after administration (Table 3).

**Table 3**

| | Intravenous administration at the dose of 0.1 mg/kg | | |
|---|---|---|---|
| | Area below the reflex amplitude linking line | Reflex amplitude (%) | |
| | | 10 min. after | 60 min. after |
| Saline | 6047 | 104.1 | 93.3 |
| TRH | 6418 | 117.7 | 79.3 |
| DN1417* | 8152 | 113.7 | 162.8 |
| Compound 1 | 8572 | 110.4 | 167.2 |
| Compound 2 | 12016 | 151.7 | 252.3 |
| Compound 5 | 11860 | 138.4 | 246.4 |
| Compound 6 | 6317 | 104.8 | 107.0 |
| Compound 7 | 7186 | 114.8 | 144.9 |
| Compound 5* | 19114 | 250.5 | 339.9 |
| Compound 6* | 10355 | 128.8 | 222.6 |
| Compound 7* | 14294 | 170.1 | 274.2 |

| | | | |
|---|---|---|---|
| * The results at the dose of 0.3 mg/kg are indicated. | | | |

## Claims (Claims for the following Contracting State(s): DE, GB, FR, IT, NL, SE, CH, LI, BE, AT, DK)

1. A peptide derivative of the following general formula [I] 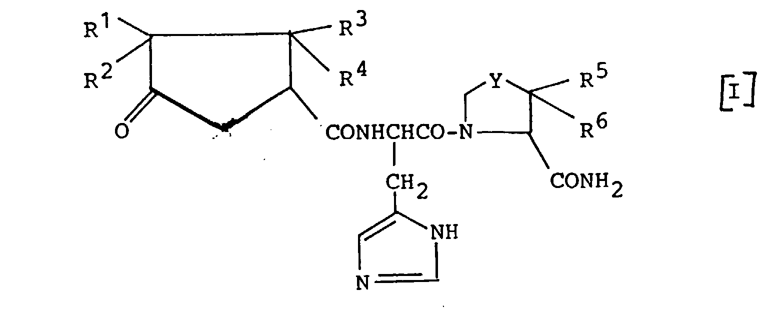 wherein R¹ and R² are the same or different and respectively mean a hydrogen atom or a C₁₋₅ alkyl group, R³ and R⁴ are the same or different and respectively mean a hydrogen atom or a C₁₋₅ alkyl or phenyl, or R³ and R⁴ combinedly mean a C₂₋₇ alkylene group, R⁵ and R⁶ are the same or different and respectively mean a hydrogen atom or a C₁₋₅ alkyl group,
and Y means -CH₂- or -S-, or an acid addition salt thereof.

2. The peptide derivative of claim 1, wherein R¹ and R² are a hydrogen atom, or an acid addition salt thereof.

3. The peptide derivative or an acid addition salt thereof as claimed in Claim 1 which is selected from N^{α}-(3-oxocyclopentanecarbonyl)-L-histidyl-L-prolinamide, N^{α}-[(1R)-3-oxocyclopentanecarbonyl]-L-histidyl-L-prolinamide, 3-[N^{α}-(3-oxocyclopentanecarbonyl)-L-histidyl]-L-thiazolidine-4-carboxamide, N^{α}-[(1R)-3-oxocyclopentanecarbonyl]-L-histidyl-3,3-dimethylprolinamide, N^{α} -(cis-2-methyl-4-oxocyclopentanecarbonyl)-L-histidyl-L-prolinamide, N^{α}-(trans-2-methyl-4-oxocyclopentanecarbonyl)-L-histidyl-L-prolinamide, N^{α}-(2,2-dimethyl-4-oxocyclopentane-carbonyl)-L-histidyl-L-prolinamide and N^{α}-(3-oxospiro[4.5] decane-1-carbonyl)-L-histidyl-L-prolinamide.

4. A method of producing a peptide derivative as claimed in Claim 1 and an acid addition salt thereof, which comprises
(A) condensing a compound of the general formula [III] 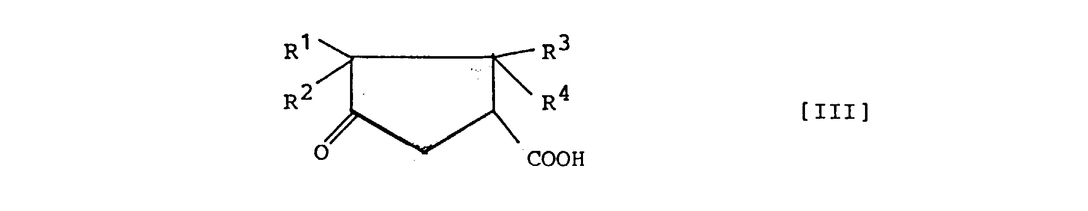 wherein R¹ - R⁴ are of the same meaning as defined in claim 1, or a reactive derivative thereof with a compound of the general formula [IV] 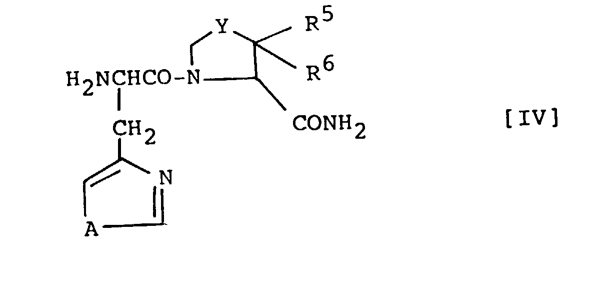 wherein A means imino group or a protected imino group, R⁵, R⁶ and Y are of the same meaning as defined in claim 1 or a salt thereof;
(B) condensing a compound of the qeneral formula [V] 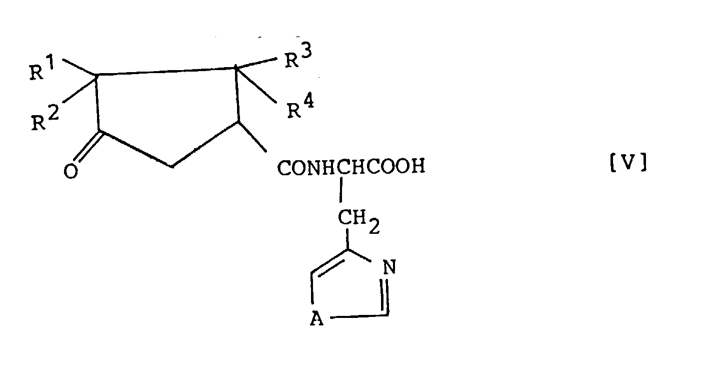 wherein R¹ - R⁴ and A are of the same meaning as defined above, or a salt thereof, or a reactive derivative thereof with an amino acid amide of the general formula [VI] 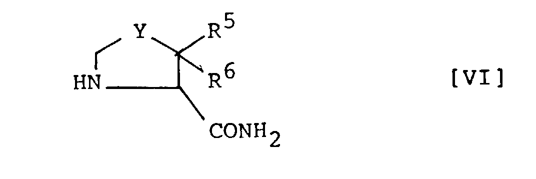 wherein Y, R⁵ and R⁶ are of the same meaning as defined above, or a salt thereof; or
(C) subjecting a compound of the general formula [VII] 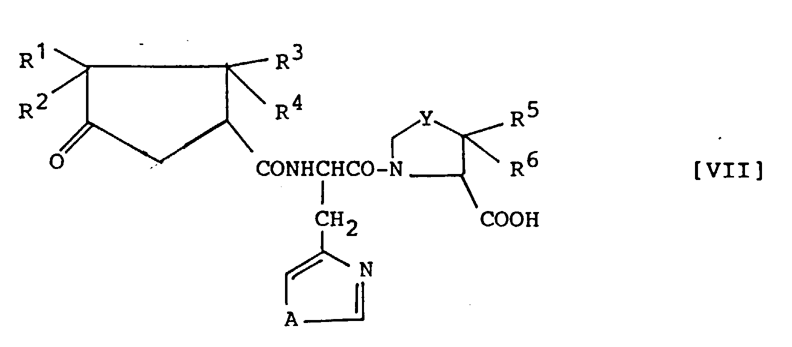 wherein R¹ - R⁶, A and Y are of the same meaning as defined above, or a salt thereof, or a reactive derivative thereof to amidation, and
(D) thereafter, when A in the product thus-obtained by the above step (A), (B) or (C) is a protected imino group, removing the protective group.

5. A pharmaceutical composition which is characterized by containing as the effective ingredient a compound as claimed in any of Claims 1, 2 or 3.

6. Use of a compound according to any of Claims 1, 2 or 3 for the preparation of a pharmaceutical composition for the treatment of central nervous disturbances.

## Claims (Claims for the following Contracting State(s): ES)

1. A method of producing a peptide derivative of the following general formula [I] 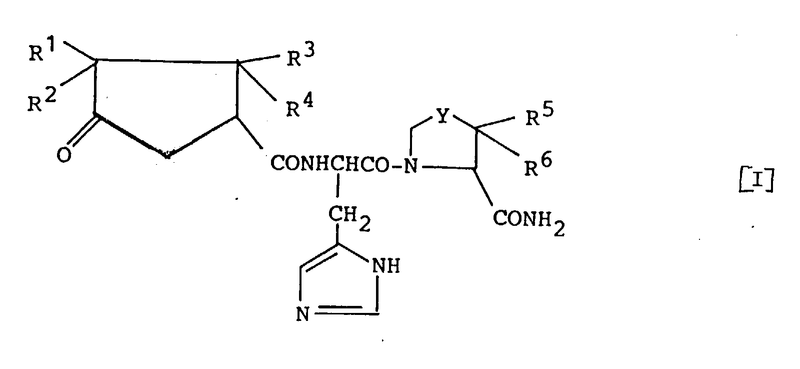 wherein R¹ and R² are the same or different and respectively mean a hydrogen atom or a C₁₋₅ alkyl group, R³ and R⁴ are the same or different and respectively mean a hydrogen atom or a C₁₋₅ alkyl or phenyl, or R³ and R⁴ combinedly mean a C₂₋₇ alkylene group, R⁵ and R⁶ are the same or different and respectively mean a hydrogen atom or a C₁₋₅ alkyl group,
and Y means -CH₂- or -S-, or an acid addition salt thereof, which method comprises
(A) condensing a compound of the general formula [III] 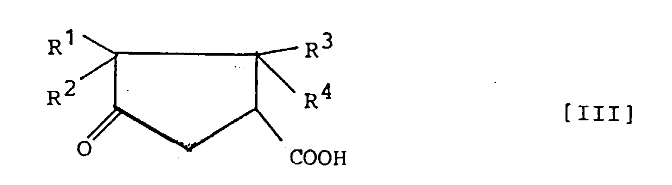 wherein R¹ - R⁴ are of the same meaning as defined above, or a reactive derivative thereof with a compound of the general formula [IV] 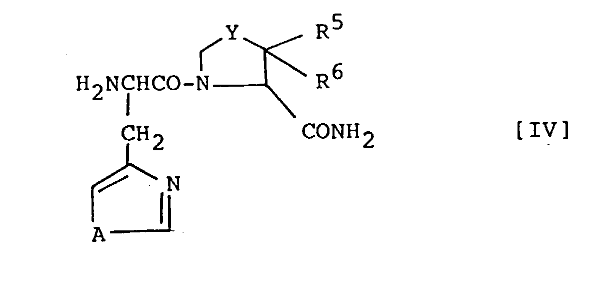 wherein A means imino group or a protected imino group, R⁵, R⁶ and Y are of the same meaning as defined above or a salt thereof;
(B) condensing a compound of the qeneral formula [V] 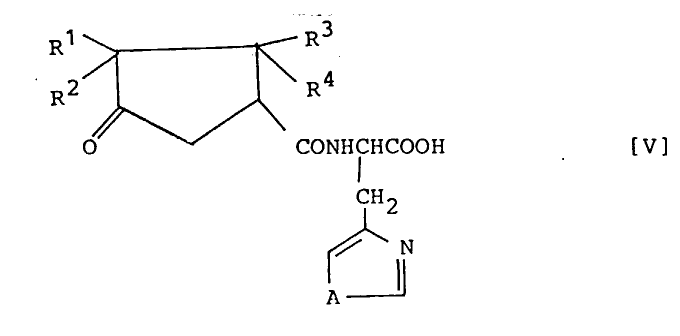 wherein R¹ - R⁴ and A are of the same meaning as defined above, or a salt thereof, or a reactive derivative thereof with an amino acid amide of the general formula [VI] 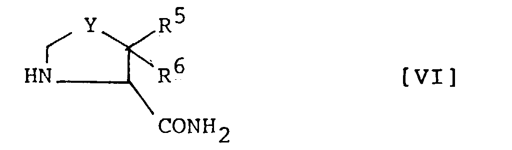 wherein Y, R⁵ and R⁶ are of the same meaning as defined above, or a salt thereof; or
(C) subjecting a compound of the general formula [VII] 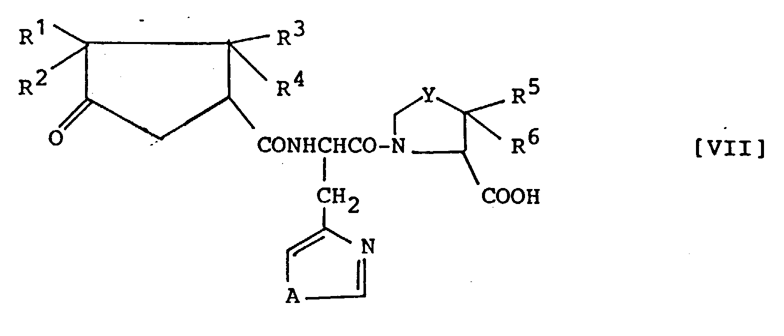 wherein R¹ - R⁶, A and Y are of the same meaning as defined above, or a salt thereof, or a reactive derivative thereof to amidation, and
(D) thereafter, when A in the product thus-obtained by the above step (A), (B) or (C) is a protected imino group, removing the protective group.

2. The method of claim 1, wherein R¹ and R² are a hydrogen atom, or an acid addition salt thereof.

3. The method of claim 1, wherein the produced peptide derrivative or acid addition salt thereof is selected from N^{α}-(3-oxocyclopentanecarbonyl)-L- histidyl-L-prolinamide, N^{α}-[(1R)-3-oxocyclopentanecarbonyl]-L-histidyl-L-prolinamide, 3-[N^{α}-(3-oxocyclopentanecarbonyl)-L-histidyl]-L-thiazolidine-4-carboxamide , N^{α}-[(1R)-3-oxocyclopentanecarbonyl]-L-histidyl-3,3-dimethylprolinamide, N^{α}-(cis-2-methyl-4-oxocyclopentanecarbonyl)-L-histidyl-L-prolinamide, N^{α}-(trans-2-methyl-4-oxocyclopentanecarbonyl)-L-histidyl-L-prolinamide , N^{α}-(2,2-dimethyl-4-oxocyclopentane-carbonyl)-L-histidyl-L-prolinamide and N^{α}-(3-oxospiro[4.5] decane-1-carbonyl)-L-histidyl-L-prolinamide.

4. A method for producing a pharmaceutical composition containing as the effective ingredient a compound as produced according to any of claims 1, 2 or 3, which method comprises admixing the compound with pharmacologically acceptable carriers, excipients and diluents.

5. The method of claim 4, wherein the produced pharmaceutical composition is useful for the treatment of central nervous disturbances.

## Patentansprüche (Patentansprüche für folgende(n) Vertragsstaat(en): AT, BE, CH, LI, DE, DK, FR, GB, IT, NL, SE)

1. Peptidderivat der folgenden allgemeinen Formel [I] 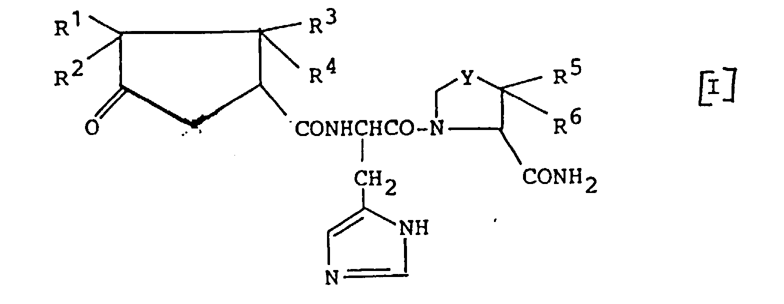 worin R¹ und R² gleich oder verschieden sind und jeweils ein Wasserstoffatom oder eine C₁₋₅-Alkylgruppe bedeuten, R³ und R⁴ gleich oder verschieden sind und jeweils ein Wasserstoffatom oder ein C₁₋₅-Alkyl oder Phenyl bedeuten oder R³ und R⁴ miteinander kombiniert eine C₂₋₇-Alkylengruppe bedeuten, R⁵ und R⁶ gleich oder verschieden sind und jeweils ein Wasserstoffatom oder eine C₁₋₅-Alkylgruppe bedeuten und Y -CH₂- oder -S- bedeutet, oder ein Säureadditionssalz davon.

2. Peptidderivat gemäß Anspruch 1, worin R¹ und R² ein Wasserstoffatom sind, oder ein Säureadditionssalz davon.

3. Peptidderivat oder Säureadditionssalz davon gemäß Anspruch 1, das ausgewählt ist aus N^{α}-(3-Oxocyclopentancarbonyl)-L-histidyl-L-prolinamid, N^{α}-[(1R)-3-Oxocyclopentancarbonyl]-L-histidyl-L-prolinamid, 3- [N^{α}-(3-Oxocyclopentancarbonyl)-L-histidyl]-L-thiazolidin-4-carboxamid, N^{α}-[(1R) -3-Oxocyclopentancarbonyl]-L-histidyl-3,3-dimethylprolinamid, N^{α} -(cis-2-Methyl-4-oxocyclopentancarbonyl)-L-histidyl-L-prolinamid, N^{α}-(trans-2-Methyl-4-oxocyclopentancarbonyl)-L-histidyl-L-prolinamid, N^{α}-(2,2-Dimethyl-4-oxocyclopentan-carbonyl)-L-histidyl-L-prolinamid und N^{α}-(3-Oxospiro[4.5]-decan-1-carbonyl)-L-histidyl-L-prolinamid.

4. Verfahren zur Herstellung eines Peptidderivats gemäß Anspruch 1 sowie eines Säureadditionssalzes davon, umfassend
(A) Kondensieren einer Verbindung der allgemeinen Formel [III] 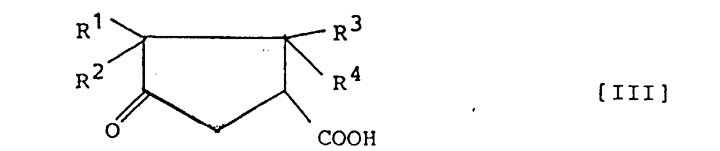 worin R¹ - R⁴ dasselbe wie in Anspruch 1 bedeuten, oder eines reaktiven Derivats davon mit einer Verbindung der allgemeinen Formel [IV] 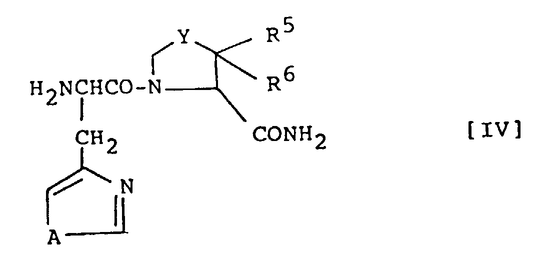 worin A eine Iminogruppe oder geschützte Iminogruppe bedeutet, R⁵, R⁶ und Y dasselbe wie in Anspruch 1 bedeuten, oder einem Salz davon;
(B) Kondensieren einer Verbindung der allgemeinen Formel [V] 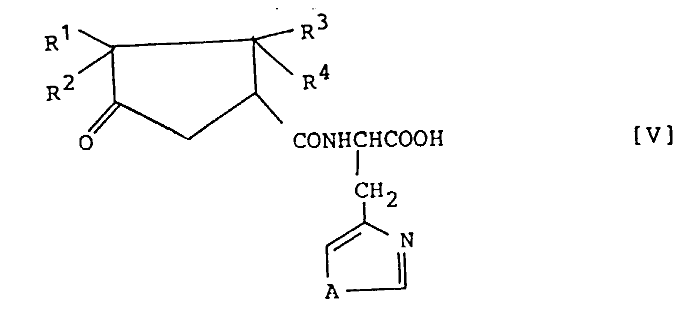 worin R¹ - R⁴ und A dasselbe wie oben bedeuten, oder eines Salzes davon oder eines reaktiven Derivats davon mit einem Aminosäureamid der allgemeinen Formel [VI] 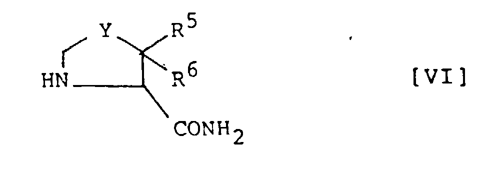 worin Y, R⁵ und R⁶ dasselbe wie oben bedeuten, oder einem Salz davon; oder
(C) Amidieren einer Verbindung der allgemeinen Formel [VII] 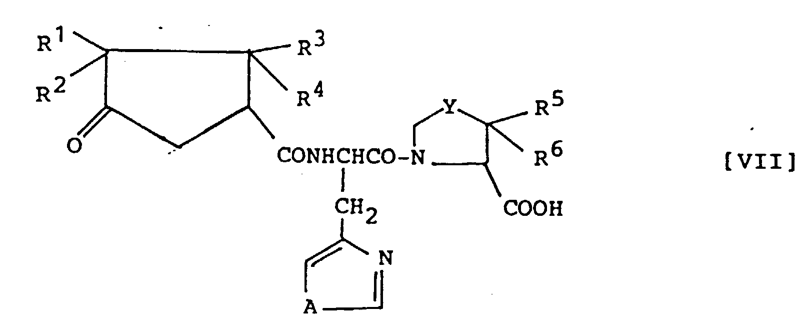 worin R¹ - R⁶, A und Y dasselbe wie oben bedeuten, oder eines Salzes davon oder eines reaktiven Derivats davon und
(D) danach, wenn A in dem durch den obigen Schritt (A), (B) oder (C) so erhaltenen Produkt eine geschützte Iminogruppe ist, Entfernen der Schutzgruppe.

5. Pharmazeutische Zusammensetzung, die dadurch gekennzeichnet ist, daß sie als Wirkstoff eine Verbindung gemäß einem der Ansprüche 1, 2 oder 3 enthält.

6. Verwendung einer Verbindung gemäß einem der Ansprüche 1, 2 oder 3 zur Herstellung einer pharmazeutischen Zusammensetzung für die Behandlung von Störungen des Zentral-nervensystems.

## Patentansprüche (Patentansprüche für folgende(n) Vertragsstaat(en): ES)

1. Verfahren zur Herstellung eines Peptidderivats der folgenden allgemeinen Formel [I] 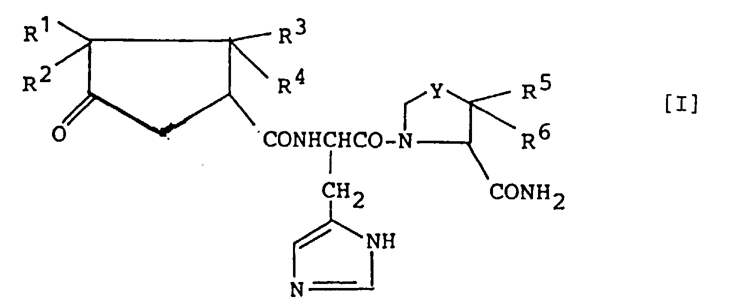 worin R¹ und R² gleich oder verschieden sind und jeweils ein Wasserstoffatom oder eine C₁₋₅-Alkylgruppe bedeuten, R³ und R⁴ gleich oder verschieden sind und jeweils ein Wasserstoffatom oder ein C₁₋₅-Alkyl oder Phenyl bedeuten oder R³ und R⁴ miteinander kombiniert eine C₂₋₇-Alkylengruppe bedeuten, R⁵ und R⁶ gleich oder verschieden sind und jeweils ein Wasserstoffatom oder eine C₁₋₅-Alkylgruppe bedeuten und Y -CH₂- oder -S- bedeutet, oder eines Säureadditionssalzes davon, wobei das Verfahren umfaßt:
(A) Kondensieren einer Verbindung der allgemeinen Formel [III] 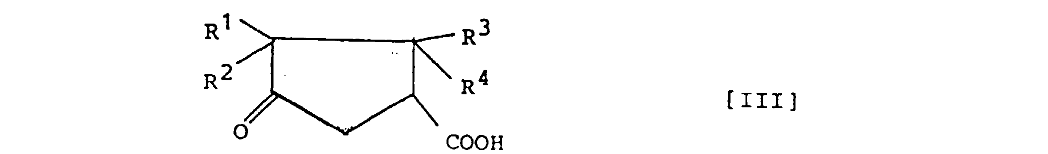 worin R¹ - R⁴ dasselbe wie oben bedeuten, oder eines reaktiven Derivats davon mit einer Verbindung der allgemeinen Formel [IV] 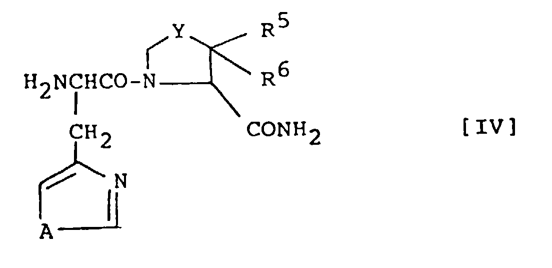 worin A eine Iminogruppe oder geschützte Iminogruppe bedeutet, R⁵, R⁶ und Y dasselbe wie oben bedeuten, oder einem Salz davon;
(B) Kondensieren einer Verbindung der allgemeinen Formel [V] 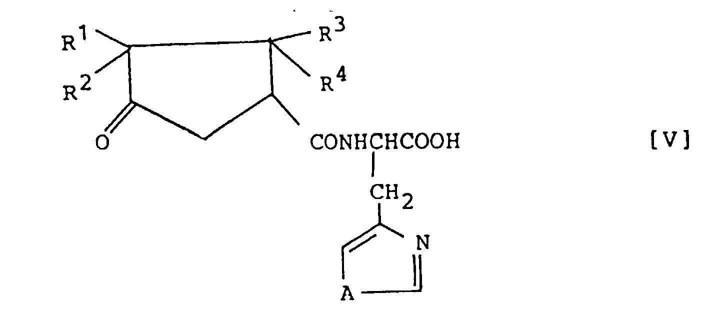 worin R¹ - R⁴ und A dasselbe wie oben bedeuten, oder eines Salzes davon oder eines reaktiven Derivats davon mit einem Aminosäureamid der allgemeinen Formel [VI] 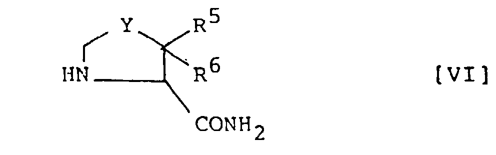 worin Y, R⁵ und R⁶ dasselbe wie oben bedeuten, oder einem Salz davon; oder
(C) Amidieren einer Verbindung der allgemeinen Formel [VII] 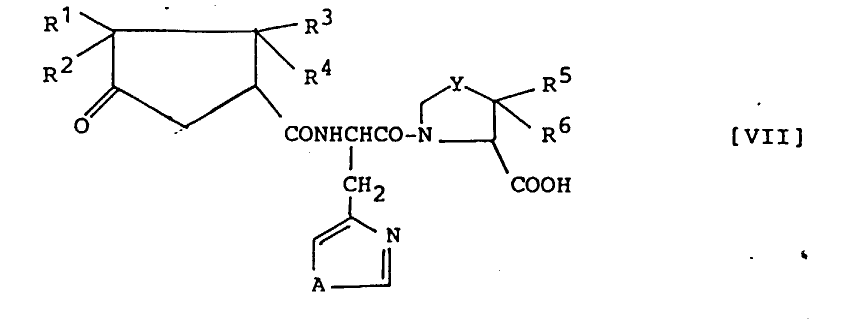 worin R¹ - R⁶, A und Y dasselbe wie oben bedeuten, oder eines Salzes davon oder eines reaktiven Derivats davon und
(D) danach, wenn A in dem durch den obigen Schritt (A), (B) oder (C) so erhaltenen Produkt eine geschützte Iminogruppe ist, Entfernen der Schutzgruppe.

2. Verfahren gemäß Anspruch 1, wobei R¹ und R² ein Wasserstoffatom sind, oder eines Säureadditionssalzes davon.

3. Verfahren gemäß Anspruch 1, wobei das hergestellte Peptidderivat oder das Säureadditionssalz davon ausgewählt ist aus N^{α}-(3-Oxocyclopentancarbonyl)-L-histidyl-L-prolinamid, N^{α}-[(1R)-3-Oxocyclopentancarbonyl] -L-histidyl-L-prolinamid, 3-[N^{α}-(3-Oxocyclopentancarbonyl)-L-histidyl]-L-thiazolidin-4-carboxamid, N^{α}-[(1R)-3-Oxocyclopentancarbonyl] -L-histidyl-3,3-dimethylprolinamid, N^{α} -(cis-2-Methyl-4-oxocyclopentancarbonyl)-L-histidyl-L-prolinamid, N^{α}- (trans-2-Methyl-4-oxocyclopentancarbonyl)-L-histidyl-L-prolinamid, N^{α}-(2,2-Dimethyl-4-oxocyclopentancarbonyl)-L-histidyl-L-prolinamid und N^{α}- (3-Oxospiro[4.5]decan-1-carbonyl) -L-histidyl-L-prolinamid.

4. Verfahren zur Herstellung einer pharmazeutischen Zusammensetzung, die als Wirkstoff eine Verbindung enthält, wie sie gemäß einem der Ansprüche 1, 2 oder 3 hergestellt wurde, wobei das Verfahren das Mischen der Verbindung mit pharmakologisch annehmbaren Trägern, Arzneimittelhilfsstoffen und Verdünnungsmitteln umfaßt.

5. Verfahren gemäß Anspruch 4, wobei sich die hergestellte pharmazeutische Zusammensetzung zur Behandlung von Störungen des Zentralnervensystems eignet.

## Revendications (Revendications pour l'(les) Etat(s) contractant(s) suivant(s): DE, GB, FR, IT, NL, SE, CH, LI, BE, AT, DK)

1. Dérivé de peptide de formule générale suivante 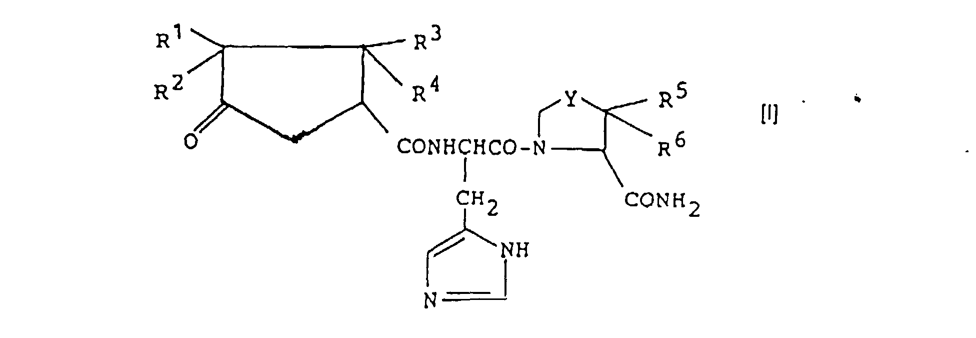 dans laquelle R¹ et R² sont identiques ou différents et signifient respectivement un atome d'hydrogène ou un groupe alkyle en C₁ à C₅, R³ et R⁴ sont identiques ou différents et signifient respectivement un atome d'hydrogène ou un groupe alkyle ou phényle en C₁ à C₅, ou R³ et R⁴ réunis signifient un groupe alkylène en C₂ à C₇, R⁵ et R⁶ sont identiques ou différents et signifient respectivement un atome d'hydrogène ou un groupe alkyle en C₁ à C₅, et Y signifie -CH₂- ou -S- ou un sel d'addition d'acide de celui-ci.

2. Dérivé de peptide selon la revendication 1, dans lequel R¹ et R² représentent un atome d'hydrogène, ou un sel d'addition d'acide de celui-ci.

3. Dérivé de peptide ou sel d'addition d'acide de celui-ci selon la revendication 1 qui est choisi parmi le N^{α}-(3-oxocyclopentanecarbonyl)-L-histidyl-L-prolinamide, le N^{α}-[(1R)-3-oxocyclopentanecarbonyl]-L-histidyl-L-prolinamide, le 3-[N^{α}-(3-oxocyclopentanecarbonyl)-L-histidyl]-L-thiazolidine-4-carboxamide, le N^{α}-[(1R)-3-oxocyclopentanecarbonyl]-L-histidyl-3,3-diméthylprolinamide, le N^{α}-(cis-2-méthyl-4-oxocyclopentanecarbonyl)-L-histidyl-L-prolinamide, le N^{α} -(trans-2-méthyl-4-oxocyclopentanecarbonyl)-L-histidyl-L-prolinamide, le N^{α}-(2,2-diméthyl-4-oxocyclopentanecarbonyl)-L-histidyl- L-prolinamide et le N^{α}-(3-oxospiro[4,5]décane-1-carbonyl)-L-histidyl-L-prolinamide.

4. Procédé de production d'un dérivé de peptide selon la revendication 1 et son sel d'adiition d'acide, qui comprend:
(A) la condensation d'un composé de formule générale [III] 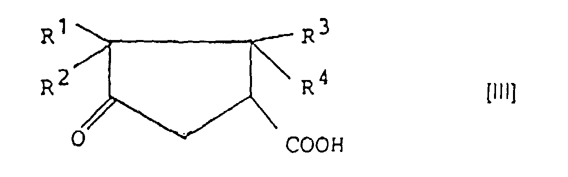 dans laquelle R¹ à R⁴ ont la même signification telle que définie dans la revendication 1, ou un dérivé réactif de celui-ci avec un composé de formule générale [IV] 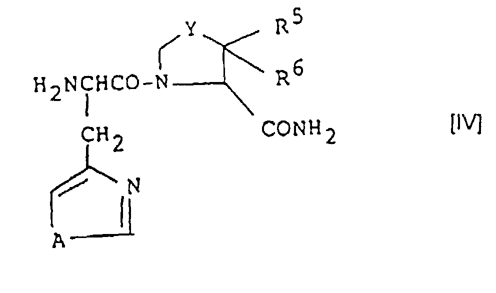 dans laquelle A signifie un groupe imino ou un groupe imino protégé, R⁵, R⁶ et Y ont la même signification telle que définie dans la revendication 1 ou un sel de celui-ci;
(B) la condensation d'un composé de formule générale [V] 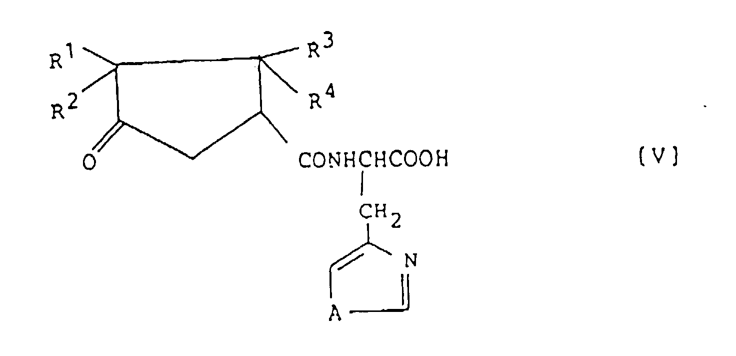 dans laquelle R¹ à R⁴ et A ont la même signification telle que définie ci-dessus, ou un sel de celui-ci, ou un dérivé réactif de celui-ci avec un amide d'acide aminé de formule générale [VI] 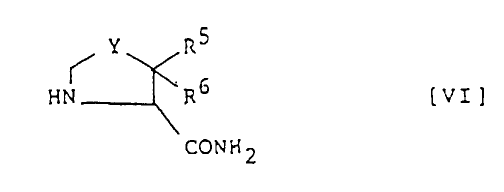 dans laquelle Y, R⁵ et R⁶ ont la même signification telle que définie ci-dessus, ou un sel de celui-ci; ou
(C) la soumission à une amidation d'un composé de formule générale [VII] 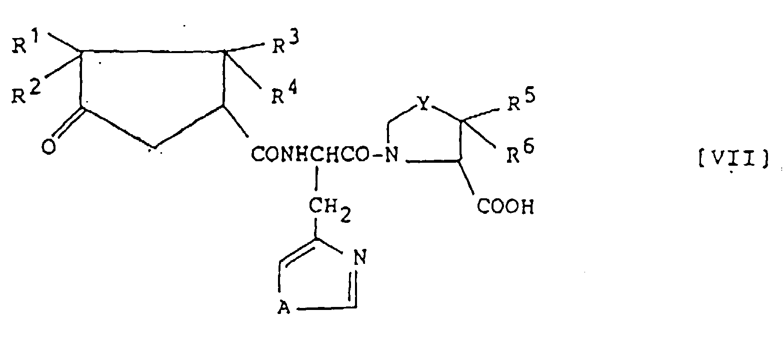 dans laquelle R¹ à R⁶, A et Y possèdent la même signification telle que définie ci-dessus, ou un sel de celui-ci, ou un dérivé réactif de celui-ci, et
(D) par la suite lorsque A dans le produit ainsi obtenu à l'aide de l'étape (A), (B) ou (C)) est un groupe imino protégé, l'élimination du groupe protecteur.

5. Composition pharmaceutique qui est caractérisée en ce qu'elle contient en tant que principe actif un composé selon l'une quelconque des revendications 1, 2 ou 3.

6. Utilisation d'un composé selon l'une quelconque des revendications 1, 2 ou 3 pour la préparation d'une composition pharmaceutique pour le traitement des troubles du système nerveux central.

## Revendications (Revendications pour l'(les) Etat(s) contractant(s) suivant(s): ES)

1. Procédé pour produire un dérivé de peptide de formule générale [I] suivante 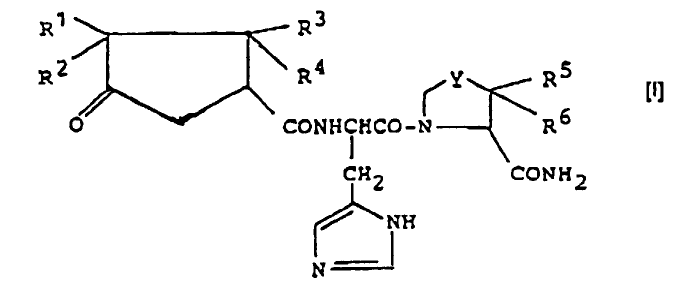 dans laquelle R¹ et R² sont identiques ou différents et signifient respectivement un atome d'hydrogène ou un groupe alkyle en C₁ à C₅, R³ et R⁴ sont identiques ou différents et signifient respectivement un atome d'hydrogène ou un groupe alkyle ou phényle en C₁ à C₅, ou R³ et R⁴ réunis signifient un groupe alkylène en C₂ à C₇, R⁵ et R⁶ sont identiques ou différents et signifient respectivement un atome d'hydrogène ou un groupe alkyle en C₁ à C₅,
et Y signifie -CH₂- ou -S- ou un sel d'addition d'acide de celui-ci, procédé qui comprend:
(A) la condensation d'un composé de formule générale [III] 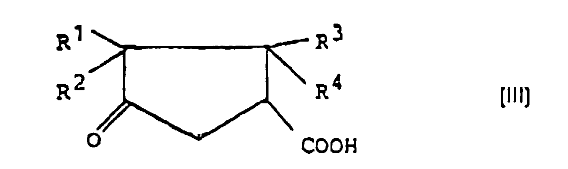 dans laquelle R¹ à R⁴ ont la même signification que définie ci-dessus, ou un dérivé réactif de celui-ci avec un composé de formule générale [IV] 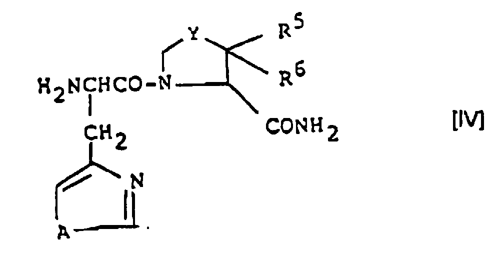 dans laquelle A signifie un groupe imino ou un groupe imino protégé, R⁵, R⁶ et Y ont la même signification que définie ci-dessus ou un sel de celui-ci;
(B) la condensation d'un composé de formule générale [V] 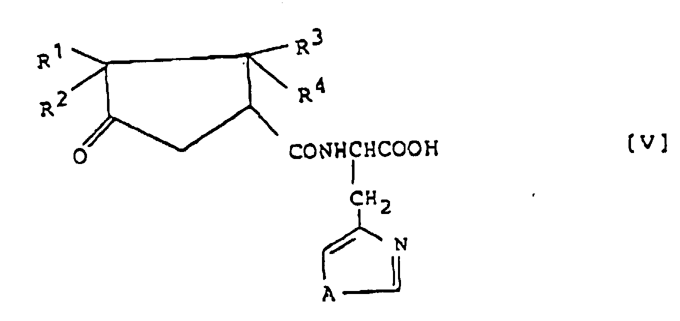 dans laquelle R¹ à R⁴ et A ont la même signification que définie ci-dessus, ou un sel de celui-ci, ou un dérivé réactif de celui-ci avec un amide d'acide aminé de formule générale [VI] 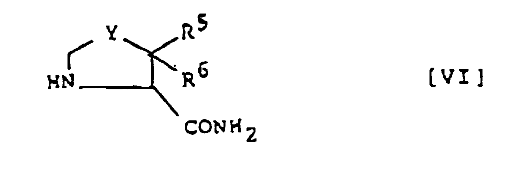 dans laquelle Y, R⁵ et R⁶ ont la même signification que définie ci-dessus, ou un sel de celui-ci; ou
(C) la soumission à une amidation d'un composé de formule générale [VII] 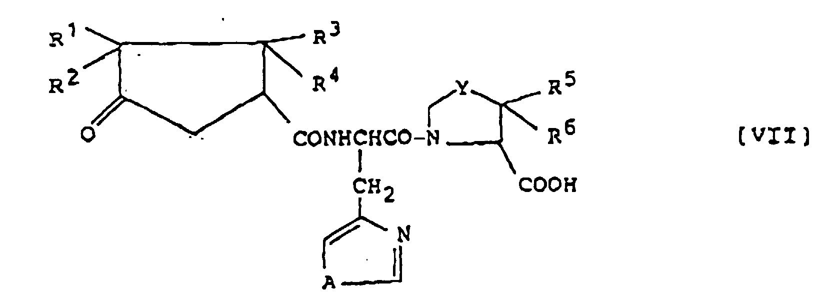 dans laquelle R¹ à R⁶, A et Y possèdent la même signification que définie ci-dessus, ou un sel de celui-ci, ou un dérivé réactif de celui-ci, et
(D) par la suite, lorsque A dans le produit ainsi obtenu à l'aide de l'étape (A), (B) ou (C) ci-dessus est un groupe imino protégé, l'élimination du groupe protecteur.

2. Procédé selon la revendication 1, dans lequel R¹ et R² représentent un atome d'hydrogène, ou un sel d'addition d'acide de celui-ci.

3. Procédé selon la revendication 1, dans lequel le dérivé de peptide produit ou un sel d'addition d'acide de celui-ci est choisi parmi:
- le N^{α}-(3-oxocyclopentanecarbonyl)-L-histidyl-L-prolinamide,
- le N^{α}-[(1R)-3-oxocyclopentanecarbonyl]-L-histidyl-L-prolinamide,
- le 3-[N^{α}-(3-oxocyclopentanecarbonyl)-L-histidyl]-L-thiazolidine-4-carboxamide,
- le N^{α} [(1R)-3-oxocyclopentanecarbonyl]-L-histidyl-3,3-diméthylprolinamide,
- le N^{α}-(cis-2-méthyl-4-oxo-cyclopentanecarbonyl]-L-histidyl-L-prolinamide,
- le N^{α}-(trans-2-méthyl-4-oxocyclopentanecarbonyl)-L-histidyl-L-prolinamide,
- le N^{α}-(2,2-diméthyl-4-oxocyclopentanecarbonyl)-L-histidyl-L-prolinamide, et
- le N^{α}-(3-oxospiro[4,5]décane-1-carbonyl)-L-histidyl-L-prolinamide.

4. Procédé de production d'une composition pharmaceutique contenant en tant que principe actif un composé produit selon l'une quelconque des revendications 1,' 2 ou 3, procédé qui consiste à mélanger le composé avec des véhicules, excipients et diluants acceptables sur le plan pharmacologique.

5. Procédé selon la revendication 4, dans lequel la composition pharmaceutique produite est utile pour le traitement des troubles du système nerveux central.
